# EUROPÄISCHE PATENTSCHRIFT

(11) **EP 0 957 083 B1**
(45) Veröffentlichungstag und Bekanntmachung des Hinweises auf die Patenterteilung: **10.04.2002**
(21) Anmeldenummer: 99108947.5
(22) Anmeldetag: 05.05.1999
(51) Int. Cl.: C07C 253/32, C07C 255/16, C07C 255/36

(54) **Verfahren zur Stabilisierung von Cyanhydrinen**
Process for stabilising cyanhydrines
Procédé de stablisation de cyanhydrines

(30) Priorität: 14.05.1998 AT 82798
(43) Veröffentlichungstag der Anmeldung: 17.11.1999
(73) Patentinhaber: DSM Fine Chemicals Austria Nfg GmbH & Co KG, 4021 Linz (AT)
(72) Erfinder: Pöchlauer, Peter, 4040 Linz (AT); Wirth, Irma, 4470 Enns (AT); Neuhofer, Rudolf, 4210 Mittertreffling (AT)
(74) Vertreter: Klostermann, Ingrid

(56) Entgegenhaltungen:
- EP-A- 0 739 870
- US-A- 4 299 843

## Beschreibung

Cyanhydrine sind etwa zur Synthese von alpha-Hydroxysäuren, alpha-Hydroxyketonen, beta-Aminoalkoholen, die zur Gewinnung biologisch wirksamer Stoffe, z. B. pharmazeutischer Wirkstoffe, Vitamine oder auch pyrethroider Verbindungen Verwendung finden, von Bedeutung.

Da Cyanhydrine an sich unbeständig sind und dazu neigen, sich in Umkehr ihrer Bildungsreaktion zu zersetzen, wurden die unterschiedlichsten Zusätze zu deren Stabilisierung verwendet. So wird in der Technik vorzugsweise Schwefelsäure und Phosphorsäure eingesetzt (Ullmanns Enzyklopädie der techn. Chemie, 4. Aufl., Bd. 7, S. 33). Aus US 4,299,843 ist die Verwendung von weiteren Säuren wie etwa HCI, Toluolsulfonsäure, Essigsäure, Propionsäure u.s.w. bekannt. Die bisher beschriebenen Säuren können jedoch für eine längere Lagerdauer oder bei Temperaturbeanspruchung der Cyanhydrine keine ausreichende Stabilisierung des Gehaltes und des Enantiomerenüberschusses gewährleisten. Aufgabe der Erfindung war es demnach, bessere Stabilisatoren zu finden.

Gegenstand der Erfindung ist ein Verfahren zur Stabilisierung von enantiomerenangereicherten Cyanhydrinen, das dadurch gekennzeichnet ist, daß dem zu stabilisierenden Cyanhydrin Zitronensäure und/oder Borsäure oder Borsäureanhydrid zugesetzt wird.
Erfindungsgemäß werden Zitronensäure undloder Borsäure oder Borsäureanhydrid in einer zur Stabilisierung ausreichenden Menge dem reinen enantiomerenangereicherten Cyanhydrin oder einer Lösung dieses Cyanhydrines zugesetzt um eine Rückbildung in HCN und die zugrundeliegende Carbonylverbindung bzw. einen Verlust an Enantiomerenüberschuß möglichst lange zu unterdrücken. Dies ist besonders während der Destillation, der Lagerung oder bei der Formulierung von großer Bedeutung. Die Menge an zugesetzter Säure kann dabei von dem zu stabilisierenden Cyanhydrin abhängen. Ein Zusatz von 0,01 bis 5 Gew. % an Säure bezogen auf das Cyanhydrin ist jedoch ausreichend. Bevorzugt wird das entsprechende Cyanhydrin mit 0,02 bis 1 Gew. % an Säure stabilisiert. Zitronensäure und Borsäure oder Borsäureanhydrid können dabei jede für sich, aber auch in Kombination in dem oben angeführten Konzentrationsbereich eingesetzt werden. Borsäureanhydrid wird bevorzugt dann eingesetzt, wenn das Cyanhydrin aus seiner Synthesereaktion möglicherweise mit Wasser verunreinigt ist. Dieses bildet dann mit Borsäureanhydrid Borsäure.

Die erfindungsgemäßen Stabilisatoren eignen sich zur Stabilisierung von enantiomerenangereicherten Cyanhydrinen, die beispielsweise durch Reaktion eines Aldehydes oder eines Ketones, eines Cyanidgruppendonors und einer Hydroxynitrillyase erhalten werden.

Unter Aldehyden sind dabei aliphatische, aromatische oder heteroaromatische Aldehyde zu verstehen. Unter aliphatischen Aldehyden sind dabei gesättigte oder ungesättigte aliphatische, geradkettige, verzweigte oder cyclische Aldehyde zu verstehen. Bevorzugte aliphatische Aldehyde sind geradkettige Aldehyde mit insbesondere 2 bis 18 C-Atomen, bevorzugt von 2 bis 12, die gesättigt oder einoder mehrfach ungesättigt sind. Der Aldehyd kann dabei sowohl C-C-Doppelbindungen als auch C-C-Dreifachbindungen aufweisen. Der Aldehyd kann unsubstituiert oder durch unter den Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Carbonsäureester-, Nitro- oder Azidogruppen substituiert sein.
Beispiele für aromatische oder heteroaromatische Aldehyde sind Benzaldehyd bzw. verschieden substituierte Benzaldehyde wie etwa 3,4-Difluorbenzaldehyd, 3-Phenoxybenzaldehyd, 4-Fluor-3-phenoxybenzaldehyd, weiters Furfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, Isophthalaldehyd u.s.w..
Ketone sind aliphatische, aromatische oder heteroaromatische Ketone, bei denen das Carbonylkohlenstoffatom ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Indolylgruppen, durch Halogen-, Ether-, Alkohol-, Acyl-, Carbonsäure-, Carbonsäureester-, Nitro- oder Azidogruppen substituiert sein.
Beispiele für aromatische oder heteroaromatische Ketone sind Acetophenon, Indolylaceton u.s.w..
Unter enantiomerenangereicherten Cyanhydrinen sind solche zu verstehen, die die (S)- oder (R)-Form in einem Anteil von größer 50%, bevorzugt größer 90% enthalten.
Bevorzugt eignen sich die erfindungsgemäßen Stabilisatoren zur Stabilisierung von aliphatischen und aromatischen, enantiomerenangereicherten Cyanhydrinen wie etwa (R)- oder (S)-3-Phenoxybenzaldehydcyanhydrin, (R)- oder (S)-4-Fluor-3-phenoxybenzaldehyd-cyanhydrin, (R)- oder (S)-3,4-Difluorbenzaldehydcyanhy-drin, (R)- oder (S)-2-Hydroxy-2,3-dimethylbutannitril, (R)- oder (S)-2-Hydroxy-2-methylpentannitril, (R)- oder (S)-2-Hydroxynonannitril, (R)- oder (S)-2-Hydroxy-2-methylphenylacetonitril (R)- oder (S)-Mandelsäurenitril.

Die zu stabilisierenden Cyanhydrine können auch als Lösung vorliegen. Lösungsmittel sind dabei solche, die auch während dem Herstellungsverfahren eingesetzt werden. Dies sind beispielsweise aliphatische oder aromatische Kohlenwasserstoffe, die gegebenenfalls halogeniert sind, Alkohole, Ether oder Ester oder Gemische davon. Bevorzugt sind Methyl-tert.Butylether (MTBE), Diisopropylether, Dibutylether, Ethylacetat oder Gemische davon.
Die erfindungsgemäßen Stabilisatoren zeichnen sich besonders bei erhöhten Temperaturen von 70 bis 110°C aus. Durch Zusatz von Zitronensäure und/oder Borsäure oder Borsäureanhydrid sinkt der Gehalt und der Enantiomerenüberschuß der (R)- oder (S)-Cyanhydrine selbst bei hohen Temperaturen und über eine längere Zeitspanne deutlich weniger ab als bei Verwendung üblicher, aus dem Stand der Technik bekannter Zusätze.

### Beispiel 1

10 g (S)-3-Phenoxybenzaldehydcyanhydrin (SCMB) wurden mit 50 mg Säure versetzt und der Gehalt sowie der Enantiomerenüberschuß bei 80°C und 100°C über eine Zeitspanne von 23 h bestimmt.
Während des Beobachtungszeitraumes wurde ständig gerührt.
Folgende Säure wurden verwendet:
Zitronensäure (H₂O-frei)
Borsäureanhydrid
sowie als Vergleich
H₂SO₄
H₃PO₄

Die Ergebnisse sind aus Tabelle 1 und 2 ersichtlich

**Tabelle 1:**

| %ee (Enantiomerenüberschuß) | | | | | | |
|---|---|---|---|---|---|---|
| Stabilisator | Zitronensäure | | Borsäureanhydrid | | H₂SO₄ | H₃PO₄ |
| Stunden | 80°C | 100°C | 80°C | 100°C | 100°C | 100°C |
| **0** | **98,6** | **98,6** | **98,6** | **98,6** | **92,6** | **92,6** |
| **1** | **98,6** | **98,6** | **98,6** | **98,6** | **87,8** | **91,8** |
| **2** | **98,6** | **98,7** | **98,6** | **98,6** | **86,8** | **92,2** |
| **3** | **98,6** | **98,7** | **98,6** | **98,6** | **86,6** | **91,5** |
| **5** | **98,6** | **98,6** | **98,7** | **98,7** | **86,7** | **90,5** |
| **6** | **98,6** | **98,6** | **98,6** | **98,7** | **-** | **-** |
| **7** | **98,5** | **98,6** | **98,6** | **98,6** | **86,6** | **89,2** |
| **8** | **98,7** | **98,6** | **98,7** | **98,6** | **87,9** | **88,4** |
| **23** | **98,7** | **98,1** | **98,7** | **98,6** | **42,1** | **70,8** |

**Tabelle 2:**

| % Gehalt | | | | | | |
|---|---|---|---|---|---|---|
| Stabilisator | Zitronensäure | | Borsäureanhydrid | | H₂SO₄ | H₃PO₄ |
| Stunden | 80°C | 100°C | 80°C | 100°C | 100°C | 100°C |
| **0** | **98** | **98** | **98** | **98** | **96,8** | **96,8** |
| **1** | **97,9** | **97,6** | **97,6** | **97,7** | **93,5** | **96,1** |
| **2** | **97,7** | **97,2** | **97,6** | **97,6** | **92,6** | **96,4** |
| **3** | **97,7** | **96,8** | **97,8** | **97,6** | **92,3** | **95,7** |
| **5** | **97,5** | **96,3** | **97,5** | **96,3** | **92,4** | **94,7** |
| **6** | **97,4** | **96** | **96,9** | **97** | **-** | **-** |
| **7** | **97,4** | **95,6** | **98** | **97** | **92,4** | **93,3** |
| **8** | **92,3** | **95,5** | **87,8** | **96,9** | **93,6** | **92,7** |
| **23** | **96,8** | **91,9** | **97,3** | **95,6** | **74,9** | **81,6** |

### Beispiel 2

Analog Bsp. 1 wurden 2 g (S)-4-Fluor-(3)-Phenoxybenzaldehydcyanhydrin mit 10 mg Säure versetzt, gerührt und der Gehalt sowie der Enantiomerenüberschuß bei 100°C bestimmt.
Als Säuren wurden wiederum Zitronensäure und Borsäureanhydrid sowie H₃PO₄ als Vergleich verwendet.
Die Ergebnisse sind aus Tabelle 3 ersichtlich.

**Tabelle 3**

| Stabilisator | Zitronensäure | | Borsäureanhydrid | | H₃PO₄ | |
|---|---|---|---|---|---|---|
| Stunden | %ee | %Gehalt | %ee | %Gehalt | %ee | %Gehalt |
| **0** | **91,1** | **97,2** | **91,1** | **97,2** | **91,1** | **97,2** |
| **1** | **91,1** | **96** | **91** | **96,3** | **91** | **96,5** |
| **2** | **91,1** | **95,1** | **91** | **95,1** | **91** | **95,8** |
| **4** | **91,1** | **93,9** | **91** | **93,3** | **91,1** | **95,1** |
| **6** | **91** | **92,4** | **91** | **91,6** | **91,1** | **93,2** |
| **7** | **91** | **91,8** | **90,9** | **91** | **91** | **91,9** |
| **8** | **90,9** | **91,2** | **90,7** | **90,2** | **91** | **90,9** |
| **24** | **87,4** | **78,5** | **88,9** | **83,8** | **84** | **76,3** |

### Beispiel 3

Analog Bsp. 1 wurden 2 g (S)-2-Hydroxynonansäurenitril mit 10 mg Citronensäure versetzt, gerührt und der Gehalt sowie. der Enantiomerenüberschuß bei 100°C bestimmt. Die Ergebnisse sind aus Tabelle 4 ersichtlich.

**Tabelle 4**

| | ohne Stabilisator | | mit Citronensäure | |
|---|---|---|---|---|
| Stunden | % ee | % Gehalt | % ee | % Gehalt |
| **0** | **93,3** | **93,8** | **93,3** | **93,8** |
| **1** | **93,2** | **95,2** | **93,2** | **96,8** |
| **3** | **92,8** | **93** | **93,3** | **94,4** |
| **4** | **91,9** | **92,4** | **93,4** | **94,7** |
| **20** | **82,5** | **91** | **93,2** | **96,5** |
| **24** | **75,2** | **90,3** | **93,2** | **95,1** |
| **44** | **45,5** | **72,8** | **91** | **94,9** |

Bei Zusatz von 10 mg H₂SO₄ oder Methansulfonsäure verfärbte sich das Nitril braun, bei Verwendung von Citronensäure als Stabilisator trat keine Verfärbung auf.

## Patentansprüche

1. Verfahren zur Stabilisierung von enantiomerenangereicherten Cyanhydrinen, **dadurch gekennzeichnet, daß** dem zu stabilisierenden Cyanhydrin Zitronensäure und/oder Borsäure oder Borsäureanhydrid als Stabilisator zugesetzt wird.

2. Verfahren nach Anspruch 1, **dadurch gekennzeichnet, daß** 0,01 bis 5 Gew.% an Stabilisator zugesetzt werden.

3. Verfahren nach Anspruch 1, **dadurch gekennzeichnet, daß** der Stabilisator dem reinen Cyanhydrin oder einer Lösung des zu stabilisierenden Cyanhydrins zugesetzt wird.

4. Verwendung von Zitronensäure und/oder Borsäure oder Borsäureanhydrid zur Stabilisierung von enantiomerenangereicherten Cyanhydrinen.

5. Verwendung von Zitronensäure und/oder Borsäure oder Borsäureanhydrid nach Anspruch 4, zur Stabilisierung von aromatischen oder aliphatischen (R)- oder (S)-Cyanhydrinen.

6. Verwendung von Zitronensäure und/oder Borsäure oder Borsäureanhydrid nach Anspruch 4, zur Stabilisierung von (R)- oder (S)-3-Phenoxybenzaldehydcyanhydrin, (R)- oder (S)-4-Fluor-3-phenoxybenzaldehydcyanhydrin, (R)- oder (S)-3,4(S)-3,4-Difluorbenzaldehydcyanhydrin, (R)- oder (S)-2-Hydroxy-2,3-dimethylbutannitril, (R)- oder (S)-2-Hydroxy-2-methylpentannitril, (R)- oder (S)-2-Hydroxynonannitril, (R)- oder (S)-2-Hydroxy-2-methylphenylacetonitril, (R)- oder (S)-Mandelsäurenitril.

## Claims

1. Process for stabilizing enantiomer-enriched cyanohydrins, **characterized in that** citric acid and/or boric acid or boric anhydride are added as a stabilizer to the cyanohydrin to be stabilized.

2. Process according to Claim 1, **characterized in that** from 0.01 to 5% by weight of stabilizer are added.

3. Process according to Claim 1, **characterized in that** the stabilizer is added to the pure cyanohydrin or to a solution of the cyanohydrin to be stabilized.

4. Use of citric acid and/or boric acid or boric anhydride for stabilizing enantiomer-enriched cyanohydrins.

5. Use of citric acid and/or boric acid or boric anhydride according to Claim 4, for stabilizing aromatic or aliphatic (R)- or (S)-cyanohydrins.

6. Use of citric acid and/or boric acid or boric anhydride according to Claim 4, for stabilizing (R)- or (S)-3-phenoxybenzaldehyde cyanohydrin, (R)- or (S)-4-fluoro-3-phenoxybenzaldehye cyanohydrin, (R)- or (S)-3,4-difluorobenzaldehyde cyanohydrin, (R)- or (S)-2-hydroxy-2,3-dimethylbutanonitrile, (R) - or (S)-2-hydroxy-2-methylpentanonitrile, (R)- or (S)-2-hydroxynonanonitrile, (R)- or (S)-2-hydroxy-2-methylphenylacetonitrile, (R)- or (S)-mandelonitrile.

## Revendications

1. Procédé de stabilisation de cyanhydrines enrichies en énantiomères, **caractérisé en ce qu'**on ajoute, comme stabilisant, de l'acide citrique et/ou de l'acide borique ou de l'anhydride borique à la cyanhydrine à stabiliser.

2. Procédé selon la revendication 1, **caractérisé en ce qu'**on ajoute de 0,01 à 5 % en poids de stabilisant.

3. Procédé selon la revendication 1, **caractérisé en ce qu'**on ajoute le stabilisant à la cyanhydrine pure ou à une solution de la cyanhydrine à stabiliser.

4. Utilisation d'acide citrique et/ou d'acide borique ou d'anhydride borique pour la stabilisation de cyanhydrines enrichies en énantiomères.

5. Utilisation d'acide citrique et/ou d'acide borique ou d'anhydride borique selon la revendication 4 pour la stabilisation de (R)- ou (S)-cyanhydrines aromatiques ou aliphatiques.

6. Utilisation d'acide citrique et/ou d'acide borique ou d'anhydride borique selon la revendication 4 pour la stabilisation de (R)- ou (S)-3-phénoxybenzaldéhyde-cyanhydrine, (R)- ou (S)-4-fluoro-3-phénoxybenzaldéhyde-cyanhydrine, (R) - ou (S)-3,4-difluorobenzaldéhyde-cyanhydrine, (R) - ou (S)-2-hydroxy-2,3-diméthylbutane-nitrile, (R) - ou (S)-2-hydroxy-2-méthylpentane-nitrile, (R)- ou (S)-2-hydroxynonane-nitrile, (R)- ou (S)-2-hydroxy-2-méthylphénylacétonitrile, (R)- ou (S)-mandélonitrile.
